# EUROPEAN PATENT APPLICATION

(11) **EP 3 175 876 A1**
(43) Date of publication of application: **07.06.2017**
(21) Application number: 15197367.4
(22) Date of filing: 01.12.2015
(51) Int. Cl.: A61M 5/315

(54) **MEDICAMENT DELIVERY DEVICE WITH USER FEEDBACK CAPABILITY**

(71) Applicant: Carebay Europe Ltd., Sliema, SLM 1643 (MT)
(72) Inventor: SÄLL, Daniel, SE-141 73 Segeltorp (SE); RENSTAD, Rasmus, 114 29 Stockholm (SE); HAUTAVIITA, Nikolaj, 19791 Bro (SE)

(57) **Abstract**

The present disclosure relates to a medicament delivery device (1) comprising: a main housing (3) arranged to accommodate a medicament container, an activation button (5) linearly displaceable from a first position relative to the main housing (3) to a second position, whereby a medicament administration procedure for expulsion of medicament from the medicament container is initiated, a first sensor (15) arranged to detect a medicament administration procedure initiated by the activation button (5), a storage unit (19) containing remaining dose data, and processing circuitry configured to update the remaining dose data based on detection of a medicament administration procedure by the first sensor (15), wherein the medicament delivery device (1) is arranged to enable presentation of the remaining dose data.

## Description

### TECHNICAL FIELD

The present disclosure generally relates to medical devices. In particular, it relates to a medicament delivery device.

### BACKGROUND

Medicament delivery devices such as injectors nowadays provide possibilities for the users themselves to handle medicament delivery in an easy, safe and reliable manner. Moreover, such devices also facilitate the administration of drugs for hospital personnel.

Medicament delivery devices may generally be divided into two classes, namely automatic medicament delivery devices and manual medicament delivery devices.

Automatic medicament delivery devices, such as auto-injectors, are typically spring-loaded and have a mechanical structure which enables essentially automatic drug administration when the spring has been unloaded.

Manual medicament delivery devices, such as manual injectors, on the other hand put greater responsibility on the user concerning drug administration because there are no mechanisms that provide the necessary automation in these devices. This means that greater care must be taken by the user to ensure that the correct dose is administered.

For multi-dose medicament delivery devices it is generally also desirable to provide some kind of visual dose indication to the user. Dose indication may be problematic in case a medicament delivery device is to be used for many small doses. The dose indication scale will have to be very fine and thus cluttered, resulting in difficulties for the user to determine the number of doses left.

### SUMMARY

In view of the above, a general object of the present disclosure is to provide a medicament delivery device that solves or at least mitigates the problems of the prior art.

There is hence provided a medicament delivery device comprising: a main housing arranged to accommodate a medicament container, an activation button linearly displaceable from a first position relative to the main housing to a second position, whereby a medicament administration procedure for expulsion of medicament from the medicament container is initiated, a first sensor arranged to detect a medicament administration procedure initiated by the activation button, a storage unit containing remaining dose data, and processing circuitry configured to update the remaining dose data based on detection of a medicament administration procedure by the first sensor, wherein the medicament delivery device is arranged to enable presentation of the remaining dose data.

With the term "medicament administration procedure" is meant mechanical interaction between internal components of the medicament delivery device resulting in linear displacement of a plunger rod, which thereby pushes out medicament from the medicament container, for example through a needle. Initiation of a medicament administration procedure hence refers to the commencement of this mechanical interaction.

By means of the medicament delivery device, the remaining dose data may for example be displayed by the medicament delivery device, or it may be sent to an external device, such as a smart device, for displaying it to a user. The remaining dose data is hence of the type that can be visualised on a display, for example in the form of a number indicating the number of remaining doses. This ensures that the dose indication will not be cluttered, no matter how many doses are to be administered by means of the medicament delivery device.

According to one embodiment the first sensor is an electromechanical switch arranged to be actuated by the activation button to thereby detect a medicament administration procedure.

One embodiment comprises a driver rotatably arranged relative to the main housing, wherein the driver is rotatable by linear movement of the activation button, a plunger rod arranged to be displaced linearly by rotation of the driver, wherein the driver is provided with at least one radial protrusion rotationally fixed relative to the driver, wherein the first sensor is arranged to detect rotational motion of the at least one radial protrusion, and wherein the processing circuitry is configured to update the remaining dose data based on the detection of rotational motion of the at least one radial protrusion.

According to one embodiment the at least one radial protrusion includes two tangentially spaced apart cam notches, wherein the cam notches have the same axial position along the driver.

According to one embodiment the cam notches are tangentially spaced apart by 180 degrees.

According to one embodiment the first sensor is an electromechanical switch arranged to be actuated by rotation of the at least one radial protrusion.

According to one embodiment the cam notches include permanent magnets, and wherein the first sensor is a reed switch.

According to one embodiment the at least one radial protrusion is a disc provided with at least one through-opening, and wherein the first sensor is an optical photo interrupter, wherein the disc extends in between the electromagnetic wave emitter and the electromagnetic wave sensor of the photo interrupter.

One embodiment comprises a proximal housing part received by the main housing and extending from a proximal end of the main housing, wherein the proximal housing part is rotatable and thereby linearly displaceable relative to the main housing, from an extended position relative to the main housing to a retracted position in which the medicament delivery device is ready for use, an activator switch, and a second sensor, wherein the activator switch is configured to be activated by movement of the proximal housing part towards the retracted position, thereby activating the second sensor, wherein the second sensor is configured to determine a current orientation of the medicament delivery device, and wherein the processing circuitry is configured to generate first orientation feedback data based on the current orientation of the medicament delivery device, which first orientation feedback data contains orientation feedback of a first medicament mixing stage, and wherein the medicament delivery device is arranged to enable presentation of the first orientation feedback data.

According to one embodiment the processing circuitry is configured to determine whether the current orientation is within an acceptable range of first orientations of the medicament delivery device during the first medicament mixing stage, wherein in case the current orientation is outside the acceptable range of first orientations the first orientation feedback data includes instructions to correct the orientation of the medicament delivery device.

This may in particular be advantageous when the medicament delivery device is of the type which includes a medicament container that contains one liquid chamber and one medicament powder chamber connected by a means of a bypass channel. The contents of these two chambers are mixed prior to administration, in a reconstitution procedure.

According to one known example of such a dual chamber medicament container, two plungers are initially displaced relative to each other and arranged inside the medicament container. One of the plungers blocks the bypass channel. The plungers may be moved linearly relative to each other by screwing the proximal housing part further into the main housing. Displacement of the plungers provides a free passage for the liquid through the bypass channel such that liquid can flow from the liquid chamber to the powder chamber.

By providing feedback regarding the orientation of the medicament delivery device, the user will be able to hold the medicament delivery device correctly during the first stage of the reconstitution process in which the needle should point upwards. If the needle points in other directions than upwards there is a risk that the drug would be pushed out through the needle during reconstitution, instead of pushing out the intended air bubble. The orientation feedback data facilitates for a user in the form of visual feedback to orient the medicament delivery device with needle pointing upwards during the first medicament mixing stage.

According to one embodiment the proximal housing part is provided with a recess, wherein the proximal housing part is arranged to receive the activation switch in the recess in the retracted position to thereby deactivate the activation switch and provide an indication to the processing circuitry that the first medicament mixing stage is completed, wherein the processing circuitry is configured to generate second orientation feedback data based on the current orientation of the medicament delivery device, which second orientation feedback data contains orientation feedback of a second medicament mixing stage, and wherein the medicament delivery device is arranged to enable presentation of the second orientation feedback data.

According to one embodiment the processing circuitry is configured to determine whether the current orientation is within an acceptable range of second orientations of the medicament delivery device during the second medicament mixing stage, wherein in case the current orientation is outside the acceptable range of second orientations the second orientation feedback data includes instructions to correct the orientation of the medicament delivery device.

The user can thereby be guided through the second medicament mixing stage, a rocking stage, of the constitution process in which the medicament delivery device is to be gently rocked to thereby adequately mix the composition and thus to complete the reconstitution procedure. The medicament delivery device can in this manner be made ready for use, ensuring that the user obtains an optimal medicament mix.

One embodiment comprises a transmitter, wherein the transmitter is configured to send the remaining dose data to a smart device.

One embodiment comprises a display unit arranged to display the remaining dose data.

According to one embodiment the display unit includes a circuit board on which a display is mounted on a top side of the circuit board, and wherein the first sensor is arranged on an underside of the circuit board.

Generally, all terms used in the claims are to be interpreted according to their ordinary meaning in the technical field, unless explicitly defined otherwise herein. All references to "a/an/the element, apparatus, component, means, etc. are to be interpreted openly as referring to at least one instance of the element, apparatus, component, means, etc., unless explicitly stated otherwise.

### BRIEF DESCRIPTION OF THE DRAWINGS

The specific embodiments of the inventive concept will now be described, by way of example, with reference to the accompanying drawings, in which:
Fig. 1a shows a perspective view of a general example of a medicament delivery device;
Fig. 1b shows a block diagram of the medicament delivery device in Fig 1a;
Fig. 2a is a longitudinal section of one variation of the medicament delivery device in Fig. 1a prior to medicament administration and post medicament administration;
Fig. 2b is a side view of the medicament delivery device in Fig. 2a, with half of the main housing removed, when the activation button is about to be released;
Fig. 2c is a side view of the medicament delivery device in Fig. 2a, with half of the main housing removed, ready for drug administration;
Fig. 3 is a perspective view of one variation of a medicament delivery device in Fig. 1a with half of the main housing removed to expose the interior of the medicament delivery device;
Fig. 4a is a side view of one variation of the medicament delivery device in Fig. 1a, with half of the main housing removed;
Fig. 4b is a perspective view of the medicament delivery device in Fig. 4a, with half of the main housing removed to expose the interior of the medicament delivery device;
Fig. 5 is a perspective view of one variation of the medicament delivery device in Fig. 1a, with half of the main housing removed to expose the interior of the medicament delivery device;
Fig. 6 is a block diagram of another example of a medicament delivery device;
Fig. 7a is a side view of a portion of the medicament delivery device, with half of the main housing removed, prior to the proximal housing part has been screwed into the main housing;
Fig. 7b shows a similar view of the medicament delivery device in Fig. 7a, however with an internal housing part removed to expose the proximal housing part that is below the internal housing part;
Fig. 8 shows the medicament delivery device in Fig. 7b when the proximal housing part has been further screwed into the main housing compared to its position in Fig. 7b;
Fig. 9a shows the retracted position of the proximal housing part, when it has been fully screwed into the main housing; and
Fig. 9b depicts the same position of the proximal housing part as shown in Fig. 9a, except that the internal housing part has been removed to expose the proximal housing part that is below the internal housing part.

### DETAILED DESCRIPTION

The inventive concept will now be described more fully hereinafter with reference to the accompanying drawings, in which exemplifying embodiments are shown. The inventive concept may, however, be embodied in many different forms and should not be construed as limited to the embodiments set forth herein; rather, these embodiments are provided by way of example so that this disclosure will be thorough and complete, and will fully convey the scope of the inventive concept to those skilled in the art. Like numbers refer to like elements throughout the description.

The present disclosure relates to a medicament delivery device of the button-activated type. The medicament delivery device could for example be a manual injector or a manual injector trainer device. The medicament delivery device is a multi-dose medicament delivery device. One medicament container, or cartridge, is thus used for multiple doses until emptied. Each dose can have a fixed, pre-determined, volume.

The medicament delivery device enables user feedback presentation during use. This user feedback includes remaining dose data which provides current information regarding the number of doses remaining. The remaining dose data can be presented on a display of the medicament delivery device and/or transmitted to another device, e.g. a smart device for presentation on that device. The medicament delivery device is therefore arranged to enable presentation of the remaining dose data. The user feedback may be presented visually, as audio or as a combination of both. To this end, the medicament delivery device includes a first sensor arranged to detect when a medicament administration procedure is initiated. Based on this detection, the medicament delivery device can update the remaining dose data, e.g. by subtracting one dose from the previously determined remaining doses.

According to one variation, the medicament delivery device is also arranged to enable presentation of orientation feedback data during a reconstitution procedure. This reconstitution procedure can include a first medicament mixing stage in which a liquid and a powder is mixed in a medicament container provided in the medicament delivery device, to obtain the medicament that is to be administered. The orientation feedback enables the user to orient the medicament delivery device in a correct manner during the first medicament mixing stage. The current orientation of the medicament delivery device is determined by means of a second sensor.

Moreover, the medicament delivery device is arranged to determine when the reconstitution stage enters a second medicament mixing stage in which the medicament delivery device is to be rocked to mix the liquid and powder as required. Orientation feedback is also provided in this second medicament mixing stage. This orientation feedback is however different from the previous orientation feedback, as it is directed to orientation feedback concerning the specific orientation or orientations of the medicament delivery device in the second medicament mixing stage, in which the user is required to gently rock the medicament delivery device. A user may thereby properly mix the medicament.

In both cases of orientation feedback the user will be provided with essentially real-time orientation information, for example in the form of correcting instructions if the orientation of the medicament delivery device is incorrect for the corresponding stage of mixing.

The orientation feedback data can be presented by a display unit that may be included in the medicament delivery device and/or transmitted to another device, e.g. a smart device for presentation on that device. The user feedback may be presented visually, as audio or as a combination of both.

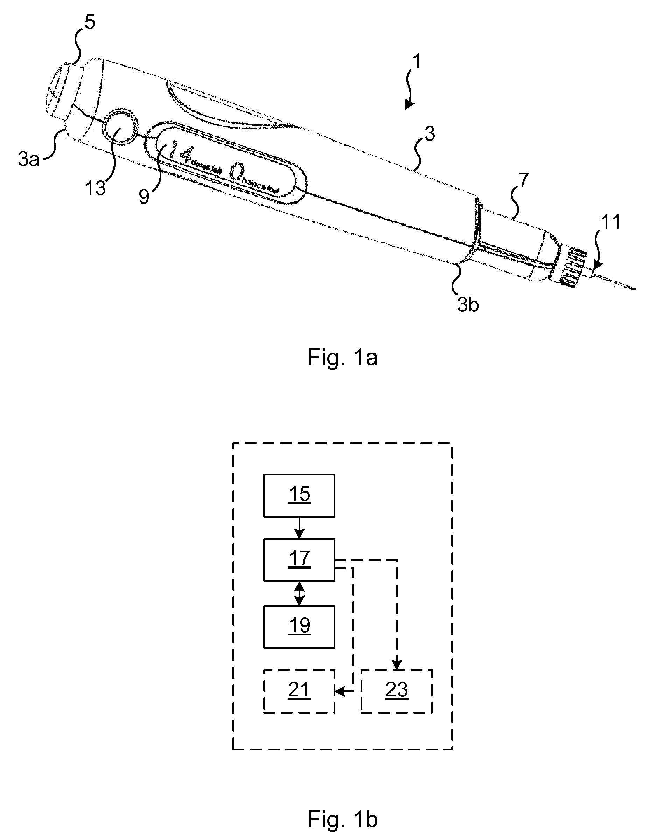

An example of a medicament delivery device 1 will now be described with reference to Figs 1a and 1b. The medicament delivery device 1 in Fig. 1a is set in a state in which it is ready to expel medicament. The medicament delivery device 1 comprises a main housing 3 arranged to accommodate a medicament container, and an activation button 5, received by the main housing 3 at a distal end 3a thereof.

The activation button 5 is linearly displaceable relative to the main housing 3, between a first position, shown in Fig. 1, and a second position. Herein, the first position will be exemplified by an extended position relative to the main housing 3 and the second position will be exemplified by a retracted position relative to the main housing 3. In the exemplified medicament delivery device 1, the activation button 5 is biased towards the extended position by means of an energy accumulation member such as a spring.

The activation button 5 is arranged to initiate medicament delivery from the medicament container by displacement of the activation button 5 from the extended position towards the retracted position. Thus, by pushing the activation button 5 into the main housing 3, the medicament administration procedure is initiated. The activation button 5 thereby interacts mechanically with at least one internal component of the medicament delivery device 1, whereby medicament delivery is commenced. Hence, when the activation button is depressed and the medicament administration procedure commences, the dose, which can be pre-determined and/or fixed, is delivered by the medicament delivery device 1.

The medicament delivery device 1 shown in Fig. 1a also has release means 13, which according to the example includes a release member including a release button displaceable in the transversal direction of the main housing 3. The activation button 5 is normally fixed in the retracted position to prevent accidental drug administration. By pressing the release button, the activation button 5 is disengaged and released from the retracted position and pushed to the extended position due to the biasing. The medicament delivery device 1 is thereby set in the state shown in Fig. 1a. Further details regarding the general construction of the medicament delivery device 1, and specifically concerning the mechanical interaction between the activation button 5 and the release means 13 is disclosed in US2013/0218098, and will therefore not be discussed in any more detail herein.

The exemplified medicament delivery device 1 comprises a proximal housing part 7 received by the main housing 3 at a proximal end 3b thereof. A needle 11 may be assembled with the proximal housing part 7. The proximal housing part 7 is rotatably arranged relative to the main housing 3. Moreover, the proximal housing part 7 is linearly displaceable relative to the main housing 3, between an extended position relative to the main housing 3 and a retracted position relative to the main housing 3, the latter being shown in Fig. 1a. This linear displacement may be obtained by rotating the proximal housing part 7 relative to the main housing 3. The proximal housing part 7 may for this purpose be provided with threads arranged to engage with corresponding threads of for example an internal housing, not shown, of the medicament delivery device 1, arranged inside the main housing 3.

Fig. 1b shows a block diagram of electronic components of the medicament delivery device 1. The medicament delivery device 1 thus comprises a first sensor 15, processing circuitry 17 and a storage unit 19. The first sensor 15 is configured to detect initiation of a medicament administration procedure. As previously described, this procedure is initiated by displacement of the activation button 5 from the extended position to the retracted position. To this end, the first sensor 15 may be configured to detect displacement of the activation button 5, from the extended position to the retracted position, or to detect displacement of some other internal component of the medicament delivery device 1, initiated by movement of the activation button 5.

The storage unit 19, e.g. a memory, contains remaining dose data concerning the number of doses remaining in the medicament container. The storage unit 19 initially stores the total number of pre-determined doses in the medicament delivery device 1. The remaining dose data hence initially contains information about the total number of pre-determined doses.

The processing circuitry 17 is configured to update the remaining dose data based on detection of a medicament administration procedure by the first sensor 15. The processing circuitry 17 hence subtracts one dose from the number of doses contained in the remaining dose data, when the first sensor 15 has detected initiation of a medicament administration procedure. Each detected or registered delivery will hence incrementally decrease the number of doses available for delivery, and this information will be included in the updated remaining dose data.

The processing circuitry 17 uses any combination of one or more of a suitable central processing unit (CPU), multiprocessor, microcontroller, digital signal processor (DSP), application specific integrated circuit (ASIC), field programmable gate arrays (FPGA) etc., capable of executing any herein disclosed operations.

The medicament delivery device 1 is arranged to enable presentation of the remaining dose data. The medicament delivery device 1 may thus according to one variation also include a display unit 21 arranged to provide visual feedback of medicament delivery device related information, such as remaining dose data. The display unit 21 may have a display 9, shown in Fig. 1a, that for example comprises electrophoretic ink (E ink). It should however be noted that the medicament delivery device ¹ does not necessarily have to include a display unit 21. As an alternative to the display unit 21, or additionally thereto, the medicament delivery device 1 could also include a transmitter configured to transmit data wirelessly to an external device such as a smart device, e.g. a smart phone or a tablet computer.

The transmitter 23 is configured to wirelessly transmit the remaining dose data generated by the processing circuitry 7. The transmitter 11 may for example be configured to send the remaining dose data to a smart phone, tablet computer or to a personal computer. The transmitter 23 includes an antenna, which may be arranged to transmit the remaining dose data over for example Bluetooth®, Wi-Fi™ or a cellular radio access network (RAN) such as Wideband Code Division Multiple Access (WCDMA) Long Term Evolution (LTE) and the 5G standard.

Alternatively, or additionally, the transmitter 23 may be configured to transmit a unique identifier of the medicament delivery device 1 and/or of the display unit 21, which in this case may be an add-on device. The display unit 21 can thus according to one variation be detachable from the main housing 3. This may in particular be useful when the medicament container has been emptied, and the medicament delivery device 1 is to be discarded. The display unit 21 may in this case be arranged to be detachable from the main housing 3 such that it can be mounted to a new medicament delivery device 1 when necessary. Advantageously, the first sensor 15, the processing circuitry 17, the storage unit 19 and the transmitter 23 are all mounted on the display unit 21, e.g. on a circuit board thereof. Transmission of the unique identifier, possibly together with other data, such as the time of drug administration may be beneficial for compliance/adherence purposes. Moreover, the display unit may be configured to display whether successful syncing with the external device has been obtained.

The processing circuitry may furthermore according to one variation be configured to generate additional dose-related data, for example the elapsed time since the previous dose. This may also be displayed on the display unit and/or transmitted to an external device.

The medicament delivery device 1 may furthermore comprise an energy storage unit. The energy storage unit may be configured to power the first sensor 15, and any other electronic component such as the processing circuitry 17, and the transmitter 23, if present. The energy storage unit may for example be a battery.

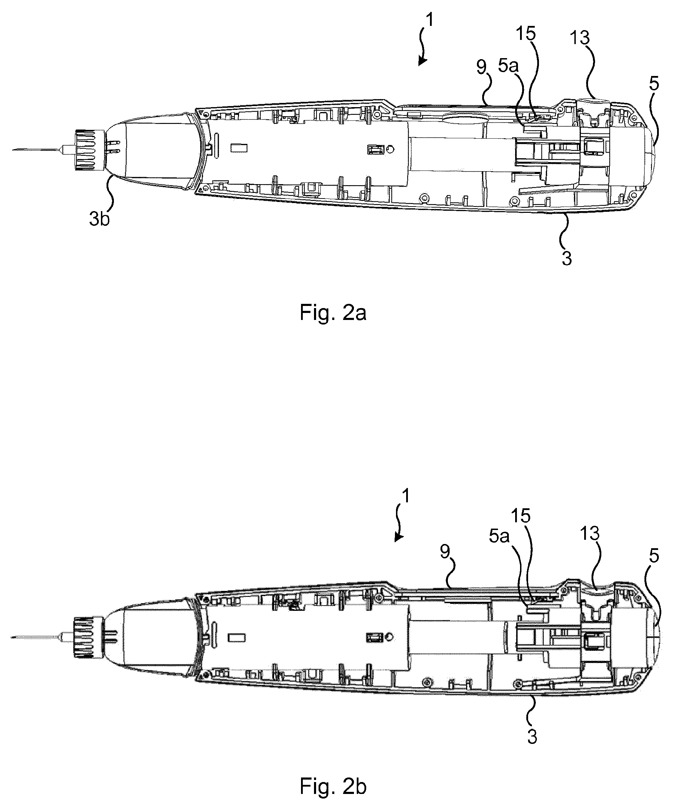

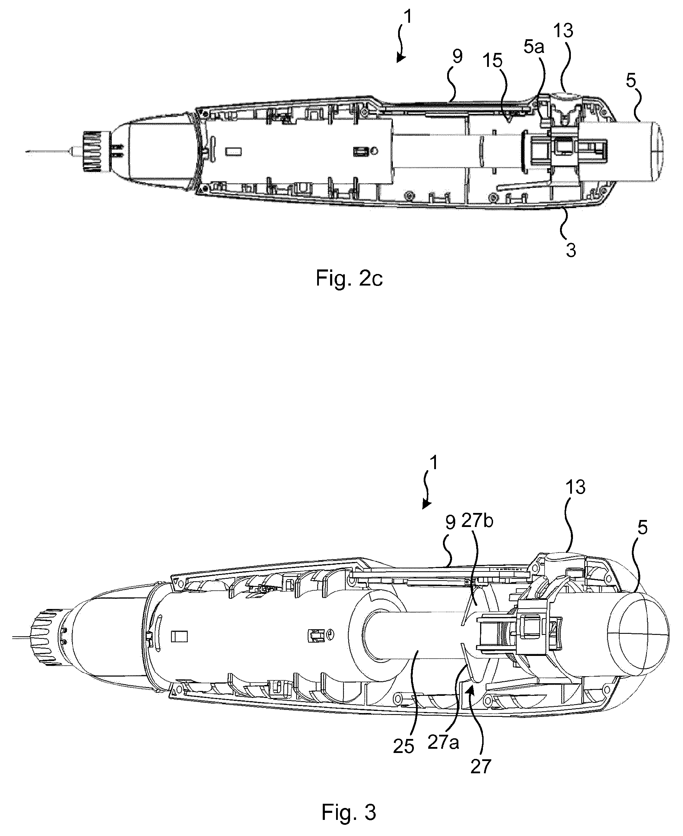

With reference to Fig. 2a a first example of an implementation of the first sensor 15 will now be described. According to this example, the first sensor 15 is an electromechanical switch arranged to be actuated by the activation button 5. The activation button 5 has a proximal end tongue 5a extending inside the main housing 3, in the longitudinal direction towards the proximal end 3b of the medicament delivery device 1. The first sensor 15 may in this case be arranged aligned with and in physical contact with the proximal end tongue 5a when the activation button 5 is in the default retracted position. The first sensor 15 may for example be mounted on an underside of a circuit board of the display unit 21. When the activation button 5 is released by the release means 13, as shown in Fig. 2b the contact between the proximal end tongue 5a and the first sensor 15 eventually ceases, as shown in Figs 2c. By pushing the activation button 5 back to the retracted position, the electromechanical switch is actuated by the new contact with the proximal end tongue 5a which is moved towards the proximal end 3b of the medicament delivery device 1. This triggers detection of the initiation of medicament administration. The processing circuitry 17 hence updates the remaining dose data accordingly. The medicament delivery device 1 then ensures that the updated remaining dose data is presented, e.g. by displaying it on the display unit 21 and/or transmitting it to an external device for presentation by the external device. The remaining dose data may for example be visualised by the number of doses left. For example, the number of doses left prior to a drug administration may be 14 doses, wherein the remaining dose data may be updated to 13 doses after the next dose has been administered.

Furthermore, upon the cease of contact between the activation button 5 and the first sensor 15, the current number of remaining doses, prior to medicament administration may be displayed by the display unit 21, or if applicable it may be transmitted to an externa device. The updated remaining dose data may then be displayed and/or transmitted when the electromechanical switch anew is actuated by the activation button 5, i.e. when moving towards the retracted positon.

Fig. 3 shows another example of an implementation of the first sensor 15. The medicament delivery device 1 may in this case be provided with a driver 25 which is arranged to be rotated by linear displacement of the activation button 5. The driver 25 is arranged to receive a plunger rod, not shown, wherein rotational motion of the driver 25 is transformed into linear motion of the plunger rod for expulsion of medicament from the medicament container. The driver 25 includes at least one radial protrusion 27.

The at least one radial protrusion 27 is rotationally fixed relative to the driver 25. The first sensor 15 is arranged to detect rotational motion of the at least one radial protrusion 27. The processing circuitry 17 is configured to update the remaining dose data based on the detection of rotational motion of the at least one radial protrusion 27.

According to the example in Fig. 3, the at least one radial protrusion 27 is in the form of two tangentially spaced apart cam notches 27a, 27b. The cam notches have the same axial position along the driver 25. According to the example, the cam notches 27a, 27b are tangentially spaced apart by 180 degrees. The exact tangential spacing between the two cam notches 27a, 27b is however dependent of the rotation angle, or the number of turns, that the driver 25 completes as it moves from the extended position to the retracted position. In the example in Fig. 3 the driver 3 is rotated half a turn in this process, and therefore two cam notches 27a, 27b tangentially spaced apart by 180 degrees is necessary to ensure detection independently of the position of the cam notches 27a, 27b relative to the first sensor 15 when medicament administration is initiated. The first sensor 15 is in this case an electromechanical switch arranged to be actuated by the cam notches 27a, 27b as they rotate and are set in physically contact with the electromechanical switch.

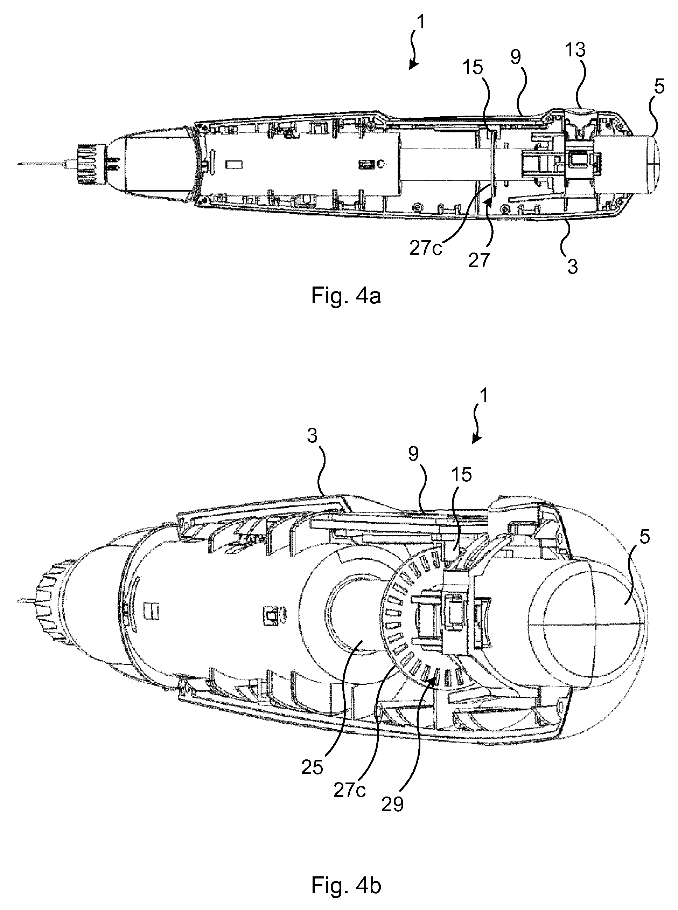

With reference to Figs 4a and 4b another example of the first sensor 15 will now be described. According to this example, the at least one radial protrusion 27 is a disc 27c provided with at least one through-opening 29. According to the example in Figs 4a and 4b the disc 27 is provided with a plurality of through-openings 29. The through-openings 29 have a radial extension and are arranged in the tangential direction around the disc 27c. The disc 27c is rotationally fixed relative to the driver 25 so that rotation of the driver 25 results in rotation of the disc 27c. The first sensor 15 is an optical photo interrupter. The disc 27c extends in between the electromagnetic wave emitter and the electromagnetic wave sensor of the photo interrupter. Detection of an electromagnetic wave propagating through the through-openings 29 triggers the processing circuitry 17 to update the remaining dose data.

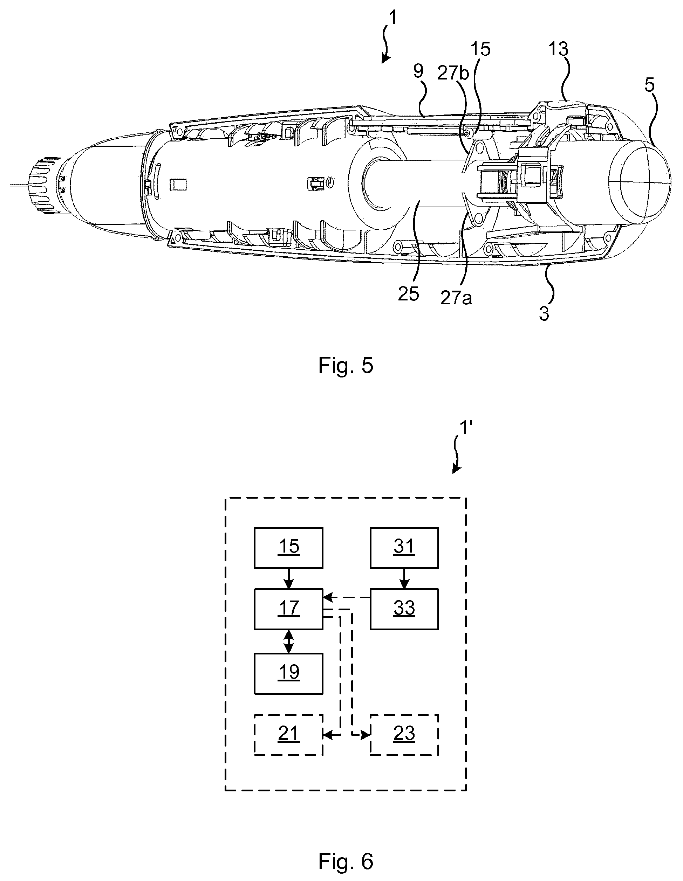

Turning now to Fig. 5 yet another example of the first sensor 15 is provided. In this case, the cam notches 25a, 25b include permanent magnets and the first sensor 15 is a reed switch. The first sensor 15 could alternatively for example be a Hall sensor or other contactless magnetic sensor.

Furthermore, according to one variation, the first sensor may be a vibration sensor, which is configured to detect characteristic vibrations induced in the medicament delivery device when manoeuvring the activation button 5 to the extended position and back to the retracted position. The vibration sensor may for example comprise a microphone or an accelerometer.

The vibrations induced during drug administration are characteristic for medicament delivery and lead to characteristic mechanical waves. The vibrations are induced by the mechanical interaction between components of the medicament delivery device. Such vibrations may for example be induced by one or more "click" sounds generated during drug administration in the case of injectors.

The processing circuitry may in this case be configured to compare a vibration measurement obtained from the vibration sensor, with a reference vibration measurement that is characteristic for the medicament delivery device during medicament delivery. In case there is a match, the processing circuitry may update the remaining dose data. Alternatively, the vibration sensor may be provided in addition to the first sensor. The first sensor, when activated according to any of the above examples, for example, by initiation of medicament administration may thus activate the vibration sensor. The vibration sensor may in this case measure the vibrations that follow, which triggers the processing circuitry to update the remaining dose data.

With reference to Fig. 6, another example of a medicament delivery device 1' is depicted. The medicament delivery device 1' is identical to medicament delivery device 1 except that it furthermore comprises an activation switch 31 and a second sensor 33. This medicament delivery device 1' is furthermore adapted to receive a medicament container that has a liquid chamber filled with liquid and a powder chamber filled with powder. The content of these two chambers is mixed prior to the first drug administration. The mixing includes a first medicament mixing stage in which the liquid flows from the liquid channel to the powder chamber. This stage requires that the medicament delivery device 1' is held with the needle pointing upwards. It furthermore includes a second medicament mixing stage in which the liquid and powder are to be mixed by rocking the medicament delivery device 1'.

The activator switch 31 is thus configured to be activated by displacement of the proximal housing part 7 towards the retracted position thereof. The second sensor 33 is thereby activated. The second sensor 31 is configured to determine a current orientation of the medicament delivery device 1'. The processing circuitry 17 is configured to generate first orientation feedback data based on the current orientation of the medicament delivery device 1'. The first orientation feedback data contains orientation feedback of a first medicament mixing stage. The medicament delivery device 1' is arranged to enable presentation of the first orientation feedback data on the display unit 21, if present, and/or to transmit the first orientation feedback data to an external device.

The processing circuitry 17 is configured to determine whether the current orientation is within an acceptable range of first orientations of the medicament delivery device during the first medicament mixing stage. In case the current orientation is outside the acceptable range of first orientations the first orientation feedback data includes instructions to correct the orientation of the medicament delivery device. The instructions may for example state to the user to direct the needle upwards.

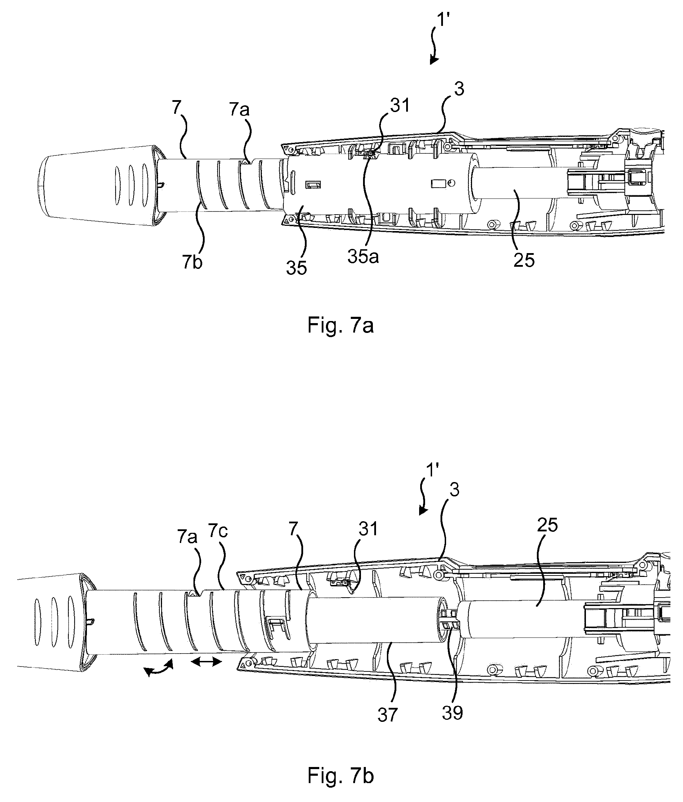

As shown in Fig. 7a the proximal housing part 7 is provided with a recess 7a. The recess 7a is arranged such that it receives the activation switch in the retracted position of the proximal housing part 7. The activation switch 31 extends radially inwards. In Fig. 7a, the proximal housing part 7a is in the extended position. In this case, the recess 7a is located outside the main housing 3. The medicament delivery device 1' may also include an internal housing 35 arranged inside the main housing 3. The internal housing 35 is arranged to receive the proximal housing part 7a and is arranged rotatably and axially fixed relative to the main housing 3.

The proximal housing part 7 may be provided with external threads 7b and the internal housing 35 may be provided with corresponding internal threads arranged to be engaged with the external threads 7b of the proximal housing part 7. The proximal housing part 7 may in this manner be screwed into the retracted position and screwed out to the extended position.

The internal housing 35 is provided with a cut-out 35 arranged radially inwards of the activation switch 31 and aligned therewith. The activation switch 35 extends into the cut-out 35.

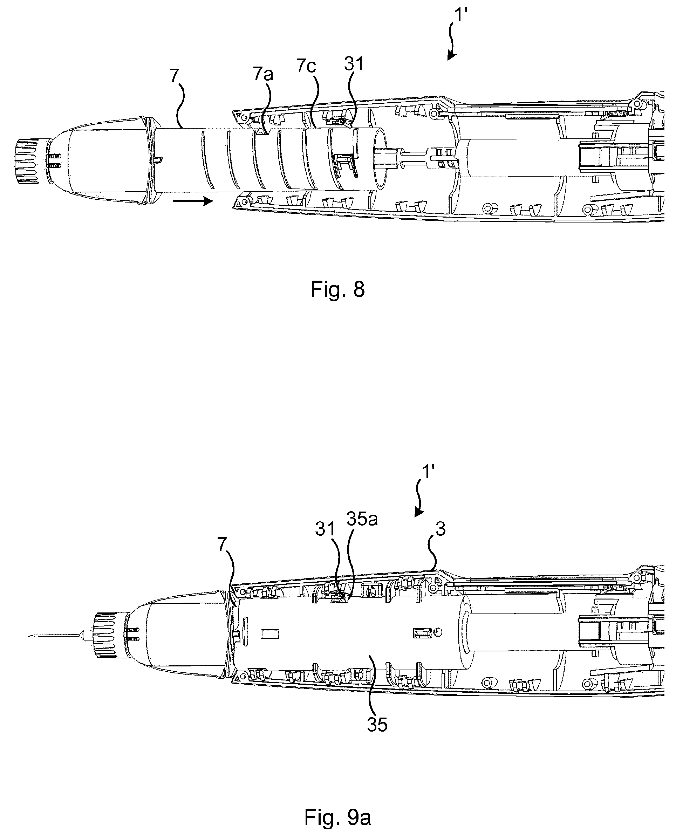

Fig. 7b shows the arrangement of the activation switch 31 relative to the proximal housing part 7, with the internal housing 35 removed for clarity. Here, the medicament container 37 and the plunger rod 39 are also exposed. By screwing the proximal housing part 7 into the main housing 3, the recess 7a is rotated and moved towards the activation switch 31. As the proximal housing part 7 is screwed into the main housing 3, the external surface 7c of the proximal housing part 7 will be provided in physical contact with the activation switch 31. This is illustrated in Fig. 8. During this time the activation switch 31 extends into the cut-out 35b of the internal housing 35, which is fixedly arranged. The activation switch 31 is thus actuated by the contact with the external surface 7c and the second sensor 33 is thereby activated and is then able to determine a current orientation of the medicament delivery device 1'.

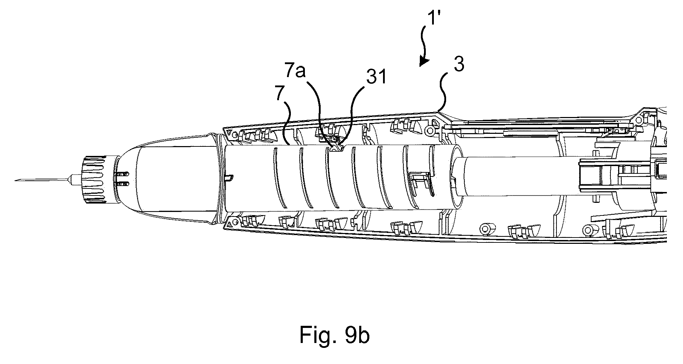

Next, when the proximal housing part 7 has obtained its retracted position, the activation switch 31 is received by the recess 7a, as shown in Fig. 9a and 9b. The activation switch 31 will then have no physical contact with the proximal housing part 7 and is thus deactivated. An indication is thereby provided to the processing circuitry 17 that the first medicament mixing stage is completed. The processing circuitry 17 is then configured to generate second orientation feedback data based on the current orientation of the medicament delivery device 1', which second orientation feedback data contains orientation feedback of the second medicament mixing stage. The medicament delivery device 1' is arranged to enable presentation of the second orientation feedback data.

The processing circuitry 17 is configured to determine whether the current orientation is within an acceptable range of second orientations of the medicament delivery device during the second medicament mixing stage. In case the current orientation is outside the acceptable range of second orientations the second orientation feedback data includes instructions to correct the orientation of the medicament delivery device. The instructions may for example state that the medicament delivery device 1' should be rocked gently.

The inventive concept has mainly been described above with reference to a few examples. However, as is readily appreciated by a person skilled in the art, other embodiments than the ones disclosed above are equally possible within the scope of the inventive concept, as defined by the appended claims.

## Claims

1. A medicament delivery device (1; 1') comprising:
a main housing (3) arranged to accommodate a medicament container (37),
an activation button (5) linearly displaceable from a first position relative to the main housing (3) to a second position, whereby a medicament administration procedure for expulsion of medicament from the medicament container (37) is initiated,
a first sensor (15) arranged to detect a medicament administration procedure initiated by the activation button (5),
a storage unit (19) containing remaining dose data, and
processing circuitry (17) configured to update the remaining dose data based on detection of a medicament administration procedure by the first sensor (15),
wherein the medicament delivery device (1; 1') is arranged to enable presentation of the remaining dose data.

2. The medicament delivery device (1; 1') as claimed in claim 1, wherein the first sensor (15) is an electromechanical switch arranged to be actuated by the activation button (5) to thereby detect a medicament administration procedure.

3. The medicament delivery device (1; 1') as claimed in claim 1, comprising a driver (25) rotatably arranged relative to the main housing (3), wherein the driver (25) is rotatable by linear movement of the activation button (5), a plunger rod (39) arranged to be displaced linearly by rotation of the driver (25), wherein the driver (25) is provided with at least one radial protrusion (27) rotationally fixed relative to the driver (25), wherein the first sensor (15) is arranged to detect rotational motion of the at least one radial protrusion (27), and wherein the processing circuitry (17) is configured to update the remaining dose data based on the detection of rotational motion of the at least one radial protrusion (27).

4. The medicament delivery device (1; 1') as claimed in claim 3, wherein the at least one radial protrusion (27) includes two tangentially spaced apart cam notches (27a, 27b), wherein the cam notches (27a, 27b) have the same axial position along the driver (25).

5. The medicament delivery device (1; 1') as claimed in claim 4, wherein the cam notches (27a, 27b) are tangentially spaced apart by 180 degrees.

6. The medicament delivery device (1; 1') as claimed in any of claims 3-5, wherein the first sensor (15) is an electromechanical switch arranged to be actuated by rotation of the at least one radial protrusion (27).

7. The medicament delivery device (1; 1') as claimed in claim 4 or 5, wherein the cam notches (27a, 27b) include permanent magnets, and wherein the first sensor (15) is a reed switch.

8. The medicament delivery device (1; 1') as claimed in claim 3, wherein the at least one radial protrusion (27) is a disc (27c) provided with at least one through-opening (29), and wherein the first sensor (15) is an optical photo interrupter, wherein the disc (27) extends in between the electromagnetic wave emitter and the electromagnetic wave sensor of the photo interrupter.

9. The medicament delivery device (1') as claimed in any of the preceding claims, comprising a proximal housing part (7) received by the main housing (3) and extending from a proximal end (3b) of the main housing (3), wherein the proximal housing part (7) is rotatable and thereby linearly displaceable relative to the main housing (3), from an extended position relative to the main housing (3) to a retracted position in which the medicament delivery device (1') is ready for use, an activator switch (31), and a second sensor (33), wherein the activator switch (31) is configured to be activated by displacement of the proximal housing part (7) towards the retracted position, thereby activating the second sensor (33), and wherein the second sensor (33) is configured to determine a current orientation of the medicament delivery device (1'), wherein the processing circuitry (17) is configured to generate first orientation feedback data based on the current orientation of the medicament delivery device (1'), which first orientation feedback data contains orientation feedback of a first medicament mixing stage, and wherein the medicament delivery device (1') is arranged to enable presentation of the first orientation feedback data.

10. The medicament delivery device (1') as claimed in claim 9, wherein the processing circuitry (17) is configured to determine whether the current orientation is within an acceptable range of first orientations of the medicament delivery device (1') during the first medicament mixing stage, wherein in case the current orientation is outside the acceptable range of first orientations the first orientation feedback data includes instructions to correct the orientation of the medicament delivery device.

11. The medicament delivery device (1') as claimed in claim 9 or 10, wherein the proximal housing part (7) is provided with a recess (7a), wherein the proximal housing part (7) is arranged to receive the activation switch (31) in the recess (7a) in the retracted position to thereby deactivate the activation switch (31) and provide an indication to the processing circuitry (17) that the first medicament mixing stage is completed, wherein the processing circuitry (17) is configured to generate second orientation feedback data based on the current orientation of the medicament delivery device (1'), which second orientation feedback data contains orientation feedback of a second medicament mixing stage, and wherein the medicament delivery device (1') is arranged to enable presentation of the second orientation feedback data.

12. The medicament delivery device (1') as claimed in claim 11, wherein the processing circuitry (17) is configured to determine whether the current orientation is within an acceptable range of second orientations of the medicament delivery device during the second medicament mixing stage, wherein in case the current orientation is outside the acceptable range of second orientations the second orientation feedback data includes instructions to correct the orientation of the medicament delivery device.

13. The medicament delivery device (1; 1') as claimed in any of the preceding claims, comprising a transmitter (23), wherein the transmitter (23) is configured to send the remaining dose data to a smart device.

14. The medicament delivery device (1; 1') as claimed in any of the preceding claims, comprising a display unit (21) arranged to display the remaining dose data.

15. The medicament delivery device (1; 1') as claimed in claim 14, wherein the display unit (21) includes a circuit board on which a display (9) is mounted on a top side of the circuit board, and wherein the first sensor (15) is arranged on an underside of the circuit board.
